# EUROPEAN PATENT APPLICATION

(11) **EP 4 299 089 A1**
(43) Date of publication of application: **03.01.2024**
(21) Application number: 22181478.3
(22) Date of filing: 28.06.2022
(51) Int. Cl.: A61M 5/158, A61M 5/32, A61M 5/34, A61M 5/14, A61M 5/31

(54) **NEEDLE ASSEMBLY FOR AN INJECTION OR INFUSION DEVICE**

(71) Applicant: Ypsomed AG, 3401 Burgdorf (CH)
(72) Inventor: Gurtner, Thomas, 3425 Koppigen (CH); Kobel, Eduard, 3456 Trachselwald (CH); Strassburger, David, 4538 Oberbipp (CH)
(74) Representative: Meier Obertüfer, Jürg

(57) **Abstract**

A needle assembly for an injection or an infusion device comprising a cannula (32) having an outer diameter, an inner diameter and a wall there between forming a lumen (49) defining a longitudinal axis connecting a proximal end to a distal end of the cannula (32). The assembly furthermore comprises a hub (33) for holding the cannula (32) wherein the hub (33) comprises a distal section (36), a proximal section (38) and a passageway (44) between the proximal and distal sections (36, 38). A holding means (37) is located in the passageway (44) between the distal and proximal sections and the holding means has an inner dimension (48) or diameter smaller than the outer diameter (47) of the cannula to form a sliding seat (45) with the cannula. The holding means (37) comprises at least one second passageway (50) connecting the proximal and distal sections of the hub.

## Description

### TECHNICAL FIELD

The current invention relates to a needle assembly for an injection or infusion device comprising a hub and a cannula.

### BACKGROUND OF THE INVENTION

Injection or infusion devices are used for the subcutaneous or intravenous (IV) delivery of a fluid medicament whereby the fluid medicament is transported from a reservoir to the human via a fluid path.

The fluid path may have one end directly connected to the reservoir and the opposite end comprises a cannula or hollow needle configured for insertion into the patient. The fluid path may comprise a tubing connecting the cannula to the reservoir. A needle holder or hub is used for holding the cannula or hollow needle and a needle insertion mechanism is used for moving the holder or hub. The needle holder is moved for needle insertion and optionally for needle retraction.

Alternatively, the fluid path may be connectable to the reservoir. During shelflife, the reservoir is fluidly disconnected from the fluid path and the connection is established just before using the injection or infusion device. The fluid path comprises a tubing having two hollow needles/cannulas at both ends and a tube connecting the hollow needles. One end is configured for insertion into the patient using the insertion and retraction mechanism and the other end is configured for insertion into the reservoir. The reservoir needle is moved by a reservoir needle insertion mechanism for penetrating, for example, a septum of the reservoir.

Alternatively, the hollow needle may be attached to a syringe for transporting fluid from the syringe to the patient. In this case the hollow needle is attached to a needle hub and the needle hub may be connected to the syringe.

The hollow needle/cannula is preferably held by the needle holder or a needle hub providing mechanical stability as the hollow needles are thin and may be damaged during fast insertion. The needle holder or hub is used for transferring the needle insertion or retraction forces to the relatively fragile hollow needle. The needle holder or hub is configured for engaging the insertion and or retraction mechanisms mentioned above.

Furthermore, the needle holder may provide a fluid connection between the tubing and the hollow needle or between the reservoir and the hollow needle. The needle hub must be constructed such that the assembly of the hub, the hollow needle and optionally the tubing is easy, safe and results in a mechanical connection that is robust and stable over time.

The connection is formed by inserting the needle into the hub having a passage adapted to receive the cannula of hollow needle. The cannula may be fixated to the hub by applying an adhesive either on one or both sides of the hub. The distal side facing the needle tip for skin insertion is preferred as adhesive present on the opposite side facing the proximal opening of the cannula may clog the entrance of the cannula.

Adding adhesive to the distal or proximal side may lead to inclusion of air bubbles thereby reducing the quality of the connection between the hub and the cannula. Assemblies between cannulas and needle hubs that eliminate air inclusions in the adhesive connection are preferred.

Additionally, inserting the cannula through the hub having a dimension of the hub locally smaller compared to the outside dimension of the cannula (thus resulting in a tight fit), may lead to skiving between an inner wall of the hub and the cannula. Particles may be formed that may contaminate the liquid medicament.

In US3186408 a needle hub is presented with a bore having an inner diameter greater than the outer diameter of the cannula. The annular space is filled with a glue that may lead to clogging of the entrance of the cannula.

In EP0438658 a needle assembly is presented for a hub and a cannula attached to the hub using an adhesive. The hub as a local section having protrusions providing for an inner dimension smaller than the outer diameter of the cannula thereby providing for an interference fit. The protrusions allow for air to escape while applying the adhesive. No precautions are taken to reduce particulate formation due to skiving and relatively thick (22 gauge) cannulas are used which lead to discomfort for the patient.

It is an object of the present invention to provide an improved needle assembly overcoming the drawbacks of the prior art needle assemblies. Furthermore a method for assembling the assembly is presented. These objectives are solved by the independent claims and specific variants are disclosed in the dependent claims.

### DEFINITIONS

The term "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The distal end or distal direction is defined by the direction of the hollow needle configured to penetrate the skin of the patient. For an injection device such as an injection pen this may be the injection needle and the end of the pen holding the needle or being configured to hold the needle is the distal end. For an infusion device the distal end and the distal direction is towards the needle configured to penetrate the skin of the patient, which may be along the axis of the device or tilted or perpendicular to the axis of the device. The distal direction in an infusion device represents the direction in which the medicament flows towards the insertion needle. The proximal direction or end is opposite to the distal direction or end.

In the claims, the word "comprising" does not exclude other elements or steps, and the indefinite article "a" or "an" does not exclude a plurality. For example "a passageway" does not exclude the fact that there may be two passageways that functionally or structurally fulfill the purpose of "a passageway". The mere fact that certain elements or steps are recited in distinct claims shall not preclude the existence of further meaningful combinations of these elements or steps.

### GENERAL DESCRIPTION OF THE INVENTION

The present invention relates to a needle assembly for an injection or an infusion device which comprises a cannula and a hub. The cannula has an outer diameter, an inner diameter and a wall there between forming a lumen defining a longitudinal axis connecting a proximal end to a distal end of the cannula. The lumen allows for flow of a liquid medicament. The hub holds or is configured to hold the cannula and comprises a distal section or distal part, a proximal section or proximal part and a passageway between the proximal and distal sections. A holding means (or holder or fixator) is located in the passageway between the distal and proximal sections. The holding means has an inner dimension or diameter smaller than the outer diameter of the cannula to form a sliding seat with the cannula and wherein the holding means comprises at least one second passageway connecting the proximal and distal sections of the hub. The second passageway is configured to allow passage of air while at least partially blocking fluid flow.

The cannula may be a hollow needle, a conduit or a duct and the cannula may be straight, and may include a curved section, a kink or a flow restriction section. The cannula may have a circular cross section but may also be non-circular such as oval or triangular cross section. One or both ends of the cannula may be sharpened optionally by a multi baffled needle tip.

The hub may be an integral part of a fluid path. Alternatively the hub may be attachable to another part such as a cartridge holder. The hub may be connectable to a syringe using, for example, a luer-lock connection. The hub may have a planar section positioned between the proximal distal sections.

The distal section may be part of the hub (thus formed integral to the hub as a monolithic part) or the distal section may be a separate part connectable to the hub but behaves functionally as one monolithic part once attached. The distal section may further comprise an entrance section facilitating entry of the cannula while assembling the needle assembly. The distal section may comprise the entrance section and an additional fixation section to be filled with an adhesive for fixating the needle to the hub.

The proximal section may be part of the (monolithic) hub or may be a separate part that is connectable to the hub and functionally behaves as a monolithic part. The proximal section may have a connector for connecting the proximal part of the needle hub with a reservoir or with an entrance/exit of the reservoir or with another fluid path. The another fluid path may comprise a second cannula. The entrance/exit of the reservoir may form the proximal section of the hub. A skirt of the hub may be part of proximal section and the skirt may be cone shaped or include a screw type of connector.

The second passageway preferably allows for air to escape either from the proximal section to the distal section or vice versa from the distal section to the proximal section when the cannula engages the holding means. The second passageway preferably prevents fluids such as medicaments or adhesives to pass through the passageway, for example, by including a flow restrictor. The distal section may comprise the entrance section and an additional fixation section to be filled with an adhesive for fixating the needle to the hub. The adhesive entering the distal section replaces the air and it is beneficial that the air can escape through the second passageway to avoid air bubbles or inclusions in the cured adhesive reducing the quality of the adhesive connection between the hub and the cannula.

Preferably the adhesive does not flow through the second passageway as it may lead to clogging of the proximal section the cannula. Therefore fluid adhesive flowing from the distal to the proximal section should be prevented or at least reduced. This can be done by one or more of the following options that may be combined with each other: The second passageway may have a dimension smaller than the outer dimension of the cannula; and/or the second passageway is straight and parallel to the axis of the cannula; or the second passageway is straight but tilted compared to the axis. Alternatively, the second passageway has a spiral shape or may include a kink connecting two straight sections. The second passageway may also include a contraction having a smaller dimension. The contraction may include a foam or membrane acting as a flow restrictor while still allowing passage of air. The second passageway may have a coating to decrease locally the surface energy for the surface of the hub thus forming a repellent surface for the adhesive.

The sliding seat may be lubricated to facilitate entrance of the cannula, for example using a fluoro-grease thereby reducing the risk of skiving and particulate formation when the proximal end of the cannula is guided through the holding means from the distal section to the proximal section thereby forming the sliding seat.

Another advantage of the second passageway may be that the proximal section may be sterilized using ETO and the ETO gas may pass between the distal and proximal sections via the second passageway.

The sliding seat allows for relative motion between the cannula and the hub. The sliding seat is based on an interference or frictional fit between the outside surface of the cannula and an inside surface of the holding means. The relative motion facilitates accurate positioning of the needle relative to the hub when assembling the assembly. The relative motion may be a strict sliding motion for positioning the cannula at a defined position, for example, for controlling the dead volume in the proximal section. Alternatively, the sliding seat allows for a tilting or rotational movement for correcting off-axis needles or deliberately introducing (and controlling) a fixed angle between the cannula and the hub.

The second passageway may be directly adj acent to an outer wall of the cannula or the second passageway may form a bypass for the sliding seat. A second passageway directly adjacent to the outer wall of the cannula may be formed by a bulge or a cut-out in the holding means. The bulge or a cut out in the holding means has locally an increased diameter or dimension compared to the outer dimension of the cannula. A bypass implies that the outside surface of the cannula is always in direct contact with an inner surface of the holding means forming a tight seal and that the additional bypass enables escapement of trapped air.

The dimension of the at least one second passageway in a plane or direction perpendicular to the longitudinal axis of the cannula is between 2% and 20% of the maximum outer diameter or dimension of the cannula. Preferably between 4% and 15%, more preferably between 6% and 10% of the maximum outer diameter or dimension of the cannula. The second passageway may have a constant dimension in the direction or plane perpendicular to the axis of the cannula, alternatively the dimension varies along the cannula and is at the maximum level at the center of the holding means and levels out, for example linearly to a minimum inner dimension of the holding means.

The dimension of the at least one second passageway in the circumferential direction of the cannula is between 10% and 50% of the outer diameter. Preferably between 20% and 40% more preferably between 25% and 35% of the outer diameter.

The dimension of the at least second passageway along the longitudinal axis of the cannula is between 20% and 200% of the maximum outer diameter or outer dimension of the cannula. Preferably between 30% and 100% of the maximum outer diameter or outer dimension of the cannula.

The at least one second passageway is arc shaped, triangular shaped or rectangular shaped in a plane perpendicular to the longitudinal axis. Alternatively, the second passageway is C-shaped or U-Shaped.

Optionally, the second passageway comprises at least one pair of opposing second passageways located in the holding means. Two passageways have the advantage that air can still escape if one second passageway gets clogged with adhesive

The needle assembly wherein the cannula is fixated by an adhesive located in the distal section of the hub, preferably a UV curable resin or epoxy resin. The adhesive is preferably entered into the distal section flowing along the outer surface of the cannula and an along an inside surface of the distal section of the hub. The resin preferably flows towards the holding means. In the meantime, air can escape from the distal section to the proximal section via the second passageway provided in the holding means. Optionally, vacuum or pressure is applied to the proximal section to control the flow of the resin.

Optionally, the distal end of the cannula is sharpened and located distally from the distal section and the proximal end has a chamfer or rounded edge and wherein the proximal end is located in the proximal section of the hub after assembling the assembly. The proximal end may be tapered and even a proximal section of the cannula may have a tapered shape. The chamfer, rounded or tapered section ensures that the proximal end does not have sharp edges and reduces the risk of particulate formation as the proximal end moves through the hub or holding means, more particularly when forming the sliding seat with the holding means. A sharp edge may lead to skiving of hub forming particles that remain within the hub. The proximal end of the cannula may be truvalized, grinded, acid-etched, laser treated, molded, over molded with another metal or polymer, blasted (for example sand blasted or solid CO2 blasted), cryogenic- or heat-treated to obtain the desired shape.

The needle assembly wherein the wall of the cannula is preferably made from stainless steel having a needle gauge between 20 and 30 and wherein the hub is made from a polymer such as a polyester, a copolyester, a polyolefin like polypropylene or polyethelyne, polystyrene, polycarbonate or the like. For example Eastman Tritan^{™} Copolyester MX731 may be used. Preferably the needle gauge is between 23 and 29. Alternatively, the cannula and the hub are both made from a polymer. The cannula is preferably made from a stiff polymer such as a PEEK. The polymers are preferably biocompatible polymers certified according to USP Class VI or ISO 10993. The surface of the distal section of the hub and or the outside surface of the cannula may be cleaned or activated prior to application of the adhesive, for example using an air plasma.

The distal section of the hub may comprise a conical entrance section facilitating the entry of the proximal end of the cannula and guiding the proximal end towards and through the holding means when assembling the assembly.

Optionally at least one cylindrical section is located between the conical section and the holding means. The cylindrical section has an inner diameter close to but greater than the outside dimension of the cannula leaving a narrow gap between the cylindrical section and the cannula. This may provide an additional flow barrier for the resin and/or provide additional adhesive strength after curing the resin.

A patch injection device comprising the needle assembly described above. The patch injection device comprises a fluid path comprising the needle assembly, a reservoir connected or connectable to or the proximal section of the assembly.

A method for assembling a needle assembly comprising the following steps:
- providing a cannula having an outer diameter, an inner diameter and a wall there between forming a lumen defining a longitudinal axis connecting a proximal end and a distal end of the cannula, the cannula having a sharp end at the distal end and preferably a rounded edge or chamfer at the proximal end,
- providing a hub for holding the cannula wherein the hub comprises a distal section a proximal section and a passageway there between and wherein the hub comprises holding means located in the passageway between the distal and proximal sections and wherein the holding means has an inner dimension or diameter smaller than the outer diameter of the cannula,
   the holding means comprises at least one second passageway connecting the proximal and distal sections of the hub,
- insertion of the proximal end of the cannula into the distal section through the holding means to form a sliding seat,
- sliding the cannula through the sliding seat to a defined cannula position,
- applying an adhesive into the distal section of the hub surrounding the cannula,
- curing the adhesive thereby fixating the cannula to the needle hub.

### LEGENDS TO THE FIGURES

While the invention has been described in detail in the drawings below and foregoing general description, such description is to be considered illustrative or exemplary and not restrictive. Variations to the disclosed embodiments can be understood and effected by those skilled in the art and practicing the claimed invention, from a study of the drawings, the disclosure, and the appended claims.
- Figure 1:: A wearable bolus injector WBI
- Figure 2:: Subunits of the wearable bolus injector
- Figure 3:: Details of the drive unit, cartridge unit and needle unit of the WBI.
- Figure 4:: Needle holder
- Figure 5:: Needle holder engaging mechanic holder
- Figure 6:: Needle holder including needle, top view
- Figure 7:: Needle holder side view
- Figure 8:: Needle holder, longitudinal section along the needle axis
- Figure 9:: Needle cross section, longitudinal section, perpendicular to the view in Figure 8
- Figure 9a: Detail Figure 9 showing holding means
- Figure 10:: Detail of the hub with two second passageways, both triangular shaped
- Figure 11:: Detail of the hub with two one passageway
- Figure 12:: Detail of the hub with a plurality of second passageways, rectangular shaped
- Figure 13:: Detail of the hub with two second passageways, both arc shaped
- Figure 14:: Detail of the hub with one square shaped opening resulting in 4 passageways
- Figure 15: Detail of the hub with one second passageway, rectangular shaped

### DETAILED DESCRIPTION OF THE FIGURES

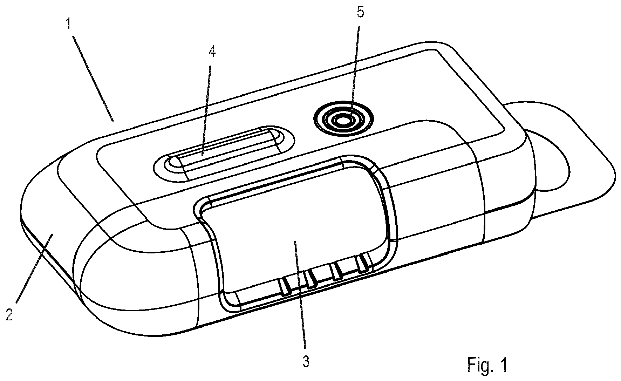

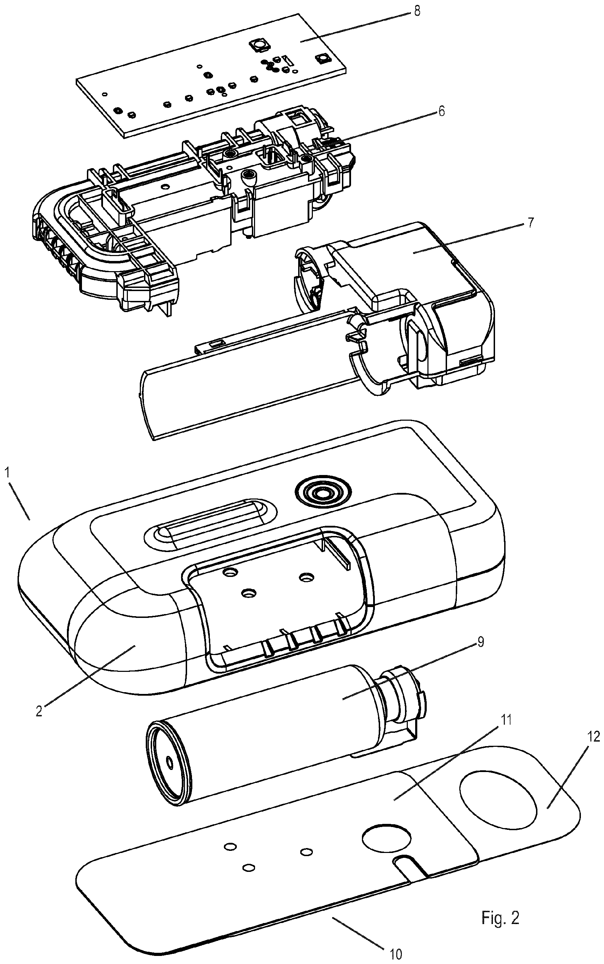

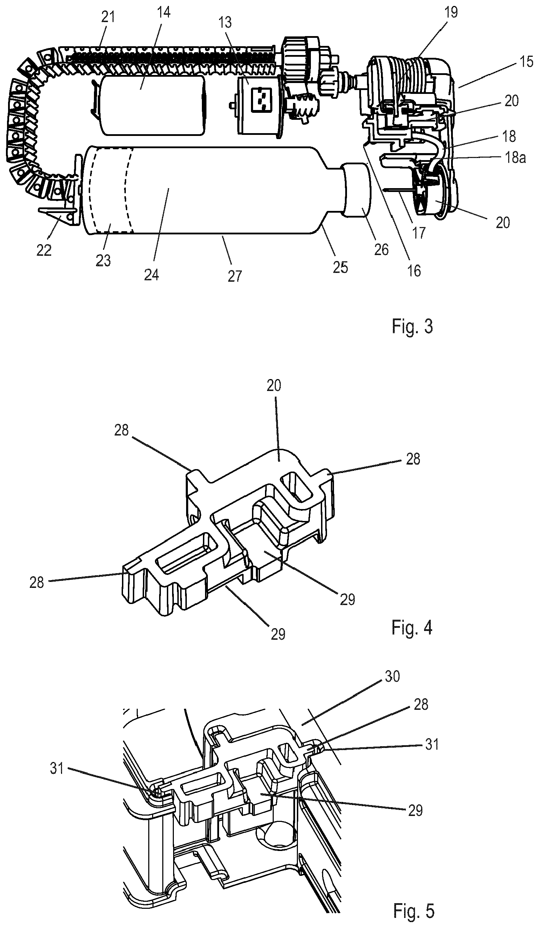

A wearable bolus injector 1 (WBI) is shown in Figure 1 as an example of an injection device. The WBI 1 is a patch injection device and may be attached to the body of a patient. The WBI comprises an outer housing 2 with a viewing window 3, a status indicator 4 and a start button 5. The outer housing 2 provides mechanical support to the inner parts or internal housing parts of the WBI and furthermore protects the inner parts from contamination. The content of a reservoir can be viewed through the viewing window 3 and the status of the device before, during after the injection is indicated by the optical status indicator 4. The status indicator 4 may be a mechanical indicator or an electrical indicator, for example using LED lights. The start button 5 may be pressed to start medicament delivery. The subunits of the WBI 1 are depicted in Figure 2 showing a drive unit 6 which is configured to advance a piston rod. Furthermore a needle unit 7 comprises and encloses a fluid path having at least one needle holder or hub, a tubing and a hollow needle or cannula attached to the needle holder. The needle unit 7 further comprises an insertion mechanism to advance and/or retract the at least one needle or cannula from a position within the needle unit to a position where a tip of the needle is outside the needle unit 7, for example outside the housing 2. An electronic control unit 8 including a printed circuit board may be part of a WBI that is electrically driven. Alternatively, the WBI is purely mechanical driven, for example driven by springs. The WBI comprises a reservoir unit 9 enclosing and protecting the liquid medicament. A patch unit 10 is used for attaching the WBI to the skin of a patient, for example using an adhesive layer 11 that is covered by a removable release liner 12. The interaction of some of the components within the needle subunit 7, the drive subunit 6 and the reservoir subunit 9 is shown in Figure 3. Members of the drive unit 6 are at least an electric motor 13 powered by a battery 14. The electric motor 13 drives an insertion mechanism 15 that is normally enclosed in a housing of the needle unit 7. The needle unit 7 further comprises a fluid path 18 comprising at least one of a hollow skin needle (or cannula) 16 and/or a cartridge needle (or cannula) 17 and a tubing 18a connecting the skin needle 16 with the cartridge needle 17. The skin needle 16 and /or the cartridge needle 17 are preferably attached to or integrated into a skin or cartridge needle holder 20. Preferably, the fluid path 18 comprises both the cartridge needle 17, the skin needle 16, the needle holder 20 and the tubing 18a. The needle holders 20 are preferably biased by at least one spring 19 acting directly or indirectly on the needle holders 20. When the spring force is released, the needle holders 20 together with one or both of the needles 16, 17 will move from a first position to a second position. In the first position the needles 16, 17 are within the needle unit 7 and in the second position at least the tip of the needle 16 is outside the needle unit 7. The skin needle 16 penetrates the skin of the patient when in the second position. The cartridge needle 17 may penetrate a septum 26 of a cartridge 27 that is part of the reservoir unit 9. The release of the spring force provided by spring 19 is controlled by the control unit 8 controlling the electric motor 13. The electric motor 13 can rotate and this rotation is transmitted via a first gearing and/or ratchet mechanism to the needle unit 7 for controlling the release of the spring 19 acting on the needle holders 20.

After insertion of the cartridge needle 17 and/or the skin needle 16, the electric motor 13 in the drive unit 6 drives a piston rod 21 towards the cartridge 27 via a second gearing mechanism. The piston rod 21 may be a segmented piston rod that can reversibly change from a linear to a curved configuration leading to space saving designs as the piston rod can be guided into a U-shape. Alternatively a straight and rigid piston rod is used. The last segment 22 may abut a piston 23 in the cartridge 27 and advancement of the piston 23 towards an outlet 25 of the cartridge 27 expels medicament 24 from the injection device via the fluid path 18. Medicament 24 can only be expelled when a septum 26 connected to the outlet 25 has been pierced prior to advancing the piston rod 21. Alternatively, the fluid path 18 is already in fluid communication with the cartridge 26 and in this alternative example there is no separate cartridge needle 17 required.

A detail of the skin needle holder 20 is presented in Figure 4. The skin needle holder 20 comprises protrusions 28 which are configured to engage guide tracks 31 configured to guide the skin needle holder, and therewith the skin needle or cannula16, when moving from the first to the second position during needle insertion (or during needle retraction). The skin needle holder 20 may comprise drive surfaces 29 used to transfer the force from the spring 19 to the skin needle holder 20 for moving the holder. A mechanic holder 30 is part of the needle unit 7 and provides mechanical support and guidance to the skin needle holder, but also to the cartridge needle holder. The skin needle holder comprises guide tracks 31, preferably linear guide tracks engaging the protrusions 28 on the skin needle holder 20 thereby guiding the skin needle holder to the inserted and/or retracted position (Figure 5).

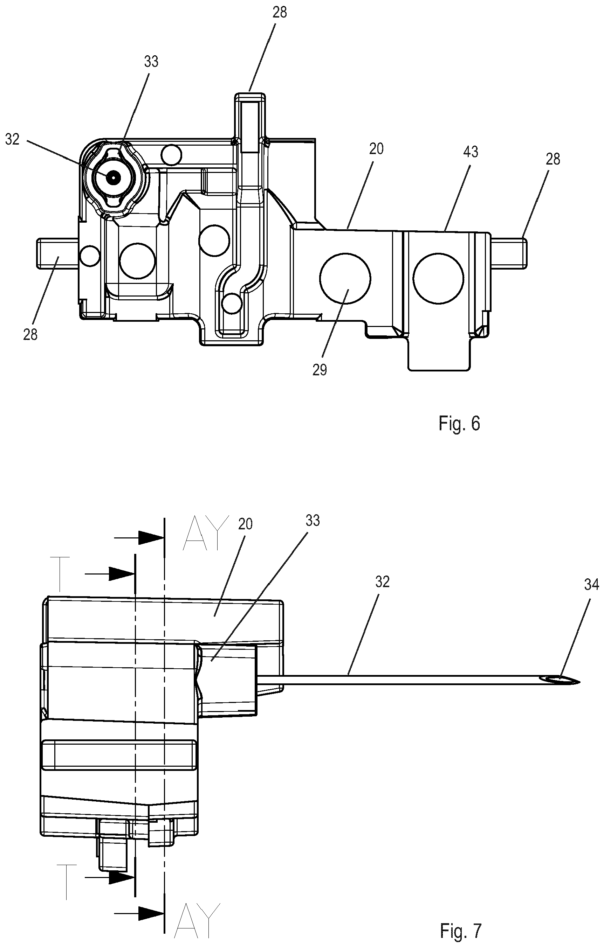

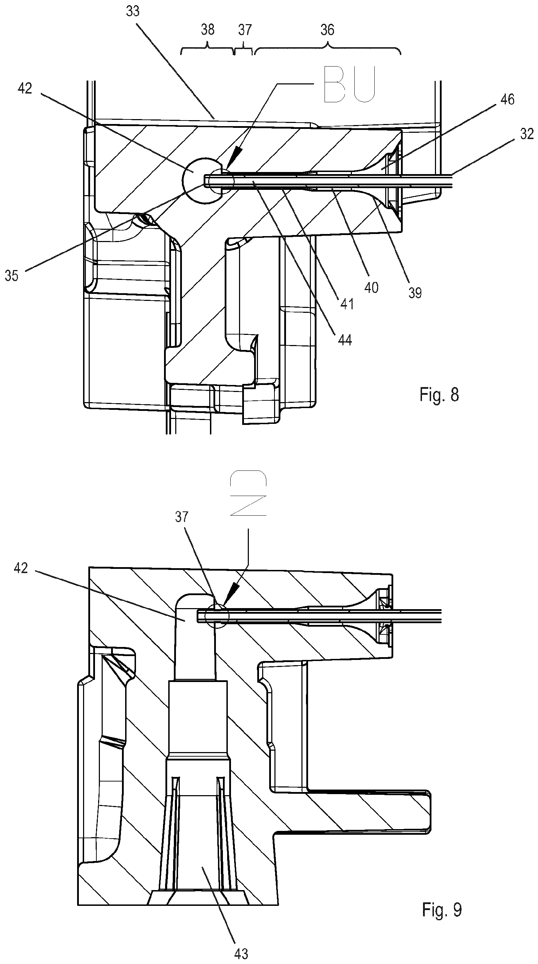

A top view and a side view of the skin needle holder 20 including a cannula or hollow needle 32 is shown in Figures 6 and 7, respectively. The needle holder 20 comprises a tube connector 43 for connecting a tubing of the fluid path 18 to the holder. Furthermore cannula 32 is enclosed by and fixated in the needle holder using hub 33. The distal tip 34 of the cannula 32 is sharpened for penetrating the skin of the patient or the septum of the reservoir. A longitudinal section along the cannula shows the details of the hub 33 (Figure 8). The hub 33 comprises a distal section 36, a proximal section 38 and a passage or opening 44 between the distal and proximal sections. The passage 44 allows for insertion and positioning of the cannula 32 during manufacturing. A holder or holding means 37 is located between the distal section 36 and the proximal section 38. The holder or holding means 37 is configured to hold the cannula 32 during manufacturing of the assembly and the dimensions of the holding means 37 are such that they constitute the narrowest part in the passage 44, and the holding means at least partially abuts the outer wall of the cannula 32 and forms an interference fit or friction fit between the hub and the cannula. The cannula 32 can be shifted, tilted or rotated for correct positioning of the cannula in the hub as a sliding seat 45 is formed between the cannula and the hub.

The distal section 36 may comprise several sub sections. For example a conical entrance section 39 facilitating entry of the proximal needle end 35 into a cylindrical guide section 40 having a smaller diameter (but still greater than the outer diameter of the cannula). The distal section may optionally have a cylindrical section 41 leaving a narrow circumferential gap between the cannula and the hub allowing for passage or air and already providing a first barrier for liquids such as an adhesive.

The proximal section 38 of the hub may be a cavity that is, for example connected to tube connector 43 for connecting the proximal end of the needle 32 with the tube 18a of the fluid path. Alternatively, the proximal section is part of a luer lock connector that may be directly attached to a syringe or syringe adapter. A hollow volume or passage 42 may be present between the holding means 37 and the tube connector 43. The proximal section 38 may also include a stop surface configured to abut the proximal end 35 of the cannula 32 thereby controlling the axial position of the cannula, and therewith the amount of dead volume present in the proximal section 38 of the hub 33.

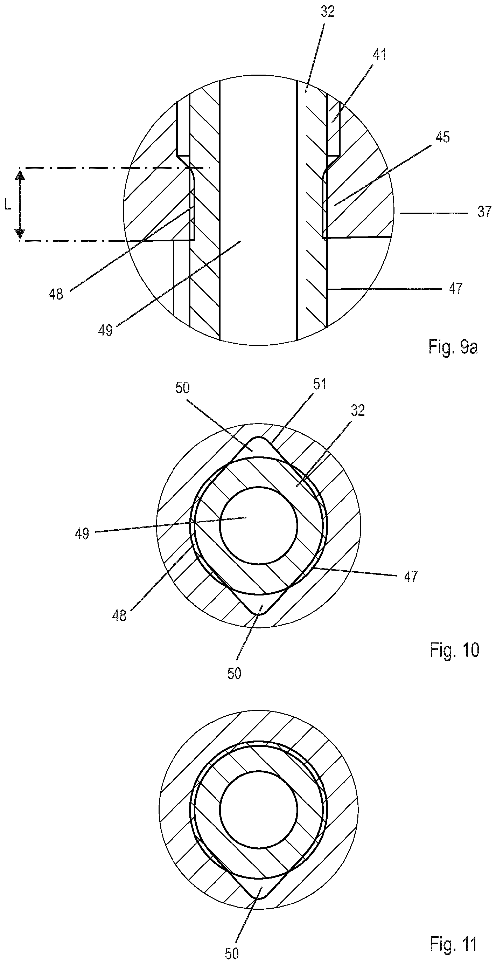

Another longitudinal section along the needle and perpendicular to Figure 8 is presented in Figure 9 showing the volume 42 for the proximal section 38 and the connection to the tube connector 43. A detailed view of the holding means 37 in Figure 9 is presented in Figure 9a. The inner diameter 48 of the narrowest part of the passage 44 is shown in the non-deformed state and the outer diameter 47 exceeds the inner diameter of the holding means 37 providing for the sliding seat 45. The length L of the holding mean is indicated and the holding means 37 is adjacent to a cylindrical section 41 of the distal section 36 of the hub 33.

Further details of the holding means 37 in a plane perpendicular to the cannula 32 are shown in Figures 10 to 15. The outside diameter 47 of the cannula 32 is greater than the inner diameter 48 of the holding means 37 thereby providing the interference fit. The medicament may flow through lumen 49 inside the cannula 32. The holding means 37 comprises two second passages 50 oriented opposite to another (Figure 10). The second passageways connect the proximal section 38 to the distal second 36 of the hub 33. The connection allows for air to escape when adhesive or resin enters the distal section of the hub. The second passages 50 may have a triangular shape 51 that locally extends from the circular shaped holding means.

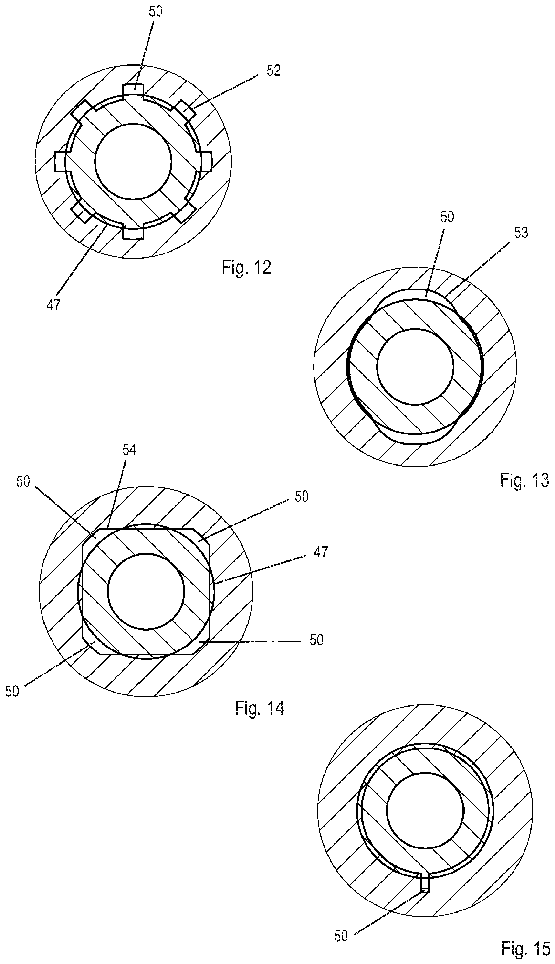

Alternatively, the holding means comprises only one second passage 50 (Figure 11, Figure 15) or a plurality of second passages 50 (Figure 12). The cross section of the passage 50 in a plane perpendicular to the longitudinal axis may be triangular shaped 51 (Figure 10), or rectangular/C-shaped 52 (Figure 12), or arc-shaped 53 (Figure 13). The holding means may have an inner shape that is square shaped 54 in its cross section (Figure 14) leaving four openings or passages 50 after insertion of the cannula 32.

The cannula 32 is secured to the hub 33 by applying an adhesive or resin 46 (Figure 8) to the hub, preferably into the conical section 39 of the distal section 36 of the hub 33. The resin will flow along the surface of the hub and along the outside surface of the cannula towards the proximal section 38. The air in front of the resin may escape through the second passageway 50 and the resin may be cured by heat and/or UV-light.

An example for the calculation of the dimensions of the second passage relative to the outer dimension of the cannula is presented in the following. A 29 gauge (Birmingham Gauge) cannula may have an outer diameter of approximately 0.3380 mm and the minimum inner diameter of the holding means may amount to 0.3150 mm, thus leading to the interference fit (approximately -0.023 mm) between the hub and the cannula. The maximum dimension of one second passage 50 in the radial direction (perpendicular to the axis) extending from the outer diameter (0.3380 mm) may have a surplus of 0.026 mm. This surplus represents 7.7% of the outer diameter of the cannula. The maximum width of one second passage in the circumferential direction of the cannula may amount to 0.10 mm which corresponds to 30% of the outer diameter of the cannula. The dimension L of the holding means 37 along the axis of the cannula may be 0.15 mm which corresponds to 45% of the outer diameter. The needle gauges used in the assembly preferably range between 23 and 33.

For a 23 Gauge needle (outer diameter is 0.6414 mm), a surplus of 0.026 mm for the second passageway in the radial direction amounts to 4% of the diameter and a width of 0.1 mm represents 16% of the diameter.

The proximal end of the cannula may have a radius between 5 % and 40% of the outer diameter of the cannula, preferably between 10% and 30% of the cannula.

**PART ANNOTATION**

| | | | |
|---|---|---|---|
| 1 | Wearable bolus injector | 30 | Housing part mechanic |
| 2 | Outer housing | | holder |
| 3 | Viewing window | 31 | Guide track |
| 4 | Status indicator | 32 | Hollow needle, cannula |
| 5 | Start button | 33 | Hub |
| 6 | Drive unit | 34 | Distal tip |
| 7 | Needle unit | 35 | Proximal end |
| 8 | Control unit | 36 | Distal section, distal part |
| 9 | Reservoir unit | 37 | Holding means, holder |
| 10 | Patch unit | 38 | Proximal section |
| 11 | Adhesive patch | 39 | Entrance section, conical |
| 12 | Release liner | | part |
| 13 | Electric motor | 40 | Entrance section guide part |
| 14 | Battery | 41 | Cylindrical section |
| 15 | Needle insertion mechanism | 42 | Volume |
| 16 | Skin needle / cannula | 43 | Tube connector |
| 17 | Cartridge needle / cannula | 44 | Passage |
| 18 | Fluid path | 45 | Sliding seat |
| 18 a | Tubing | 46 | Adhesive, resin |
| 19 | Spring | 47 | Outside diameter cannula |
| 20 | Skin needle holder /cartridge | 48 | Inner diameter holding |
| | needle holder - hub | | means |
| 21 | Piston rod | 49 | Lumen |
| 22 | Last segment | 50 | Second passage |
| 23 | Piston | 51 | Triangular shape |
| 24 | Medicament | 52 | Rectangular shape |
| 25 | Outlet | 53 | Arc shape |
| 26 | Crimp/septum | 54 | Square shaped opening |
| 27 | Cartridge | L | Length of holding means |
| 28 | Protrusion | | |
| 29 | Drive surface | | |

## Claims

1. A needle assembly for an injection or an infusion device comprising:
a cannula (32) having an outer diameter, an inner diameter and a wall there between forming a lumen (49) defining a longitudinal axis connecting a proximal end to a distal end of the cannula (32),
a hub (33) for holding the cannula (32) wherein the hub (33) comprises a distal section (36), a proximal section (38) and a passageway (44) between the proximal and distal sections (38, 36),
holding means (37) located in the passageway (44) between the distal and proximal sections (38, 36) and wherein the holding means (37) has an inner dimension or diameter (48) smaller than the outer diameter (47) of the cannula (32) to form a sliding seat (45) with the cannula (32),
**characterized in that** the holding means (37) comprises at least one second passageway (50) connecting the proximal and distal sections (38, 36) of the hub (33).

2. The needle assembly according to claim 1 wherein the sliding seat (45) allows for relative motion between the cannula (32) and the hub (33).

3. The needle assembly according to claim 2 wherein the second passageway (50) is directly adjacent to an outer wall of the cannula (32) or wherein the second passageway is a bypass for the sliding seat (45).

4. The needle assembly according the claim 3, wherein the dimension of the at least one second passageway (50) in a direction perpendicular to the longitudinal axis is between 2% and 20% of the outer diameter (47).

5. The needle assembly according to claims 3 or 4, wherein the dimension of the at least one second passageway (50) in the circumferential direction of the cannula is between 10 % and 40% of the outer diameter (47).

6. The needle assembly according the claim 5, wherein the dimension L of the at least second passageway (50) along the longitudinal axis of the cannula is between 20% and 200% of the outer diameter (47).

7. The needle assembly according the any of the previous claims, wherein the at least one second passageway (50) is arc shaped or rectangular shaped in a plane perpendicular to the longitudinal axis.

8. The needle assembly according any of the previous claims, wherein at least one pair of opposing second passage ways (50) is located in the holding means (37).

9. The needle assembly according to any of the previous claims, wherein the cannula (32) is fixated by an adhesive (46) located in the distal section (36) of the hub (33), preferably a UV curable resin or epoxy resin.

10. The needle assembly according to any of the previous claims, wherein the distal end of the cannula (32) is sharpened and located distally from the distal section (36) and wherein the proximal end has a chamfer or rounded edge and wherein the proximal end is located in the proximal section (38).

11. The needle assembly according to any of the previous claims, wherein the cannula (32) is made from stainless steel having a needle gauge between 20 and 33 and wherein the hub (33) is made from a polymer.

12. The needle assembly according to any of the previous claims wherein the distal section (36) of the hub (33) comprises a conical entrance (39).

13. The needle assembly according to claim 12 wherein at least one cylindrical section (41) is located between the conical section (39) and the holding means (37).

14. A patch injection device comprising the needle assembly according to any of the previous claims.

15. A method for assembling a needle assembly according to claims 1 to 13 comprising the following steps:
providing a cannula (32) having an outer diameter, an inner diameter and a wall there between forming a lumen (49) defining a longitudinal axis connecting the proximal end and the distal end of the cannula (32),
the cannula (32) having a sharp end at the distal end and a rounded edge or chamfer at the proximal end,
providing a hub (33) for holding the cannula (32) wherein the hub (33) comprises a distal section (36) a proximal section (38) and a passageway 44) there between and wherein the hub (33) comprises holding means (37) located in the passageway (44) between the distal and proximal sections (36, 38) and wherein the holding means (37) has an inner dimension or diameter (48) less than the outer diameter (47) of the cannula (32),
the holding means (37) comprises at least one second passageway (50) connecting the proximal and distal sections (38, 36) of the hub (33),
insertion of the proximal end of the cannula into the distal section (36) through the holding means (37) to form a sliding seat (45),
sliding the cannula (32) through the sliding seat (45) to a defined cannula position, applying an adhesive (46) into the distal section (36) of the hub surrounding the cannula, curing the adhesive (46) thereby fixating the cannula (32) to the hub (33).
